# EUROPEAN PATENT SPECIFICATION

(11) **EP 1 487 515 B1**
(45) Date of publication and mention of the grant of the patent: **12.03.2014**
(21) Application number: 03745540.9
(22) Date of filing: 18.03.2003
(51) Int. Cl.: A61L 31/18, A61L 31/14

(54) **MRI AND X-RAY COMPATIBLE STENT MATERIAL**
MRT- UND RÖNTGENKOMPATIBLES STENTMATERIAL
MATERIAU DE STENT COMPATIBLE AUX RAYONS X ET A L'IRM

(30) Priority: 22.03.2002 US 63125
(43) Date of publication of application: 22.12.2004
(60) Divisional application: 10158668.3
(73) Proprietor: Boston Scientific Limited, St. Michael, Barbados, West Indies (BB)
(72) Inventor: JANSEN, Lex P., Pleasanton, CA 94566 (US); GRIFFIN, Stephen, Sunnyvale,CA 94086 (US)
(74) Representative: Peterreins, Frank
(86) International application number: PCT/US2003/008337
(87) International publication number: WO 2003/082362

(56) References cited:
- WO-A-93/19803
- WO-A-95/30384
- US-A- 5 344 402
- US-A- 6 137 060

## Description

### Background of Invention

Stents may be placed within the body to maintain and/or treat a body lumen that has been impaired or occluded. Stents are typically made from strands of material formed into a tube, of rolled sheets of material with openings or of tubes with material removed therefrom to form a stent pattern. Stents are usually delivered into the body lumen using a catheter. The catheter carries the stent to the desired site and the stent is released from the catheter and expands to engage the inner surface of the lumen. Placement of the stent may be monitored by fluoroscopy where the stent includes radiopaque materials.

Stents may be balloon expandable, self-expanding or a combination of self-expanding and balloon expandable. Self-expanding stents are made with a material having a restoring force. Suitable materials for self-expanding stents include spring steel and shape memory materials such as Nitinol. Balloon expandable stents are often made with elastic, plastically deformable materials such as stainless steel. Other suitable materials for stents include cobalt chrome alloys such as elgiloy, tantalum and other plastically deformable metals, platinum/tungsten alloys and titanium alloys.

Because stents are delivered through often tortuous vessels, they must be flexible. In order to achieve flexible and expandable stents, the stent material desirably should have a high modulus of elasticity. At the same time, the materials should be sufficiently radiopaque to be visible under fluoroscopy. Moreover, with the widespread use of Magnetic Resonance Imaging (MRI), it is desirable for the stent material to be MRI compatible so as not to distort any magnetic resonance images that may be taken of the body in the region of the stent.

Similar material considerations also apply to other types of medical implants, including, for example, vena cava filters as well as other implant devices which require high strength as well as resistance to fracture so that the device may be deployed without fracturing.

There remains a need for stent materials and other implant materials which have high modulii of elasticity, are radiopaque and which are MRI compatible.

WO 95/30384 discloses a stent formed of multiple filaments arranged in two sets of oppositely directed helical windings interwoven with one another in a braided configuration. Each of the filaments is a composite including a central core and a case surrounding the core. In an embodiment, the core is formed of a tungsten/rhenium alloy. Favorable mechanical characteristics of the stent are determined by the case, while the core enables in vivo imaging of the stent.

US patent number 6,137,060 discloses a multifilament wire including two or more strands of nickel-titanium (NiTi) alloy wire which are twined about a higher density core wire. The multifilament wire may be used as wires of a medical stent. In an embodiment, the core wire comprises a tungsten/rhenium alloy.

Without limiting the scope of the invention a brief summary of some of the claimed embodiments of the invention is set forth below. Additional details of the summarized embodiments of the invention and/or additional embodiments of the invention may be found in the Detailed Description of the Invention below.

A brief abstract of the technical disclosure in the specification is provided as well for the purposes of complying with 37 C.F.R. 1.72.

### Summary of Invention

In one embodiment, the invention is directed to a medical implant for implantation in a bodily vessel where the implant comprises a tungsten-rhenium alloy. The implant has a flow passage therethrough and, desirably, is in the form of a stent or vena cava filter. The implant is manufactured from a tungsten-rhenium alloy consisting essentially of tungsten and rhenium. Typically, tungsten will be present in an amount ranging from about 75 weight percent to about 99 weight percent and rhenium will be present in an amount ranging from about 25 weight percent to about 1 weight percent. The implant may be manufactured from a sheet of material or from a tube or from other suitable forms of the alloy.

Additional details and/or embodiments of the invention are discussed below.

### Brief Description of Drawings

FIG. 1 is a perspective view of an implant in the form of a stent.
FIG. 2 is a perspective view of the stent of FIG. 1 in an expanded state.
FIG. 3 is a side view of another implant in the form of a stent.
FIG. 4 is a flat pattern of the stent of FIG. 3.

### Detailed Description

While this invention may be embodied in many different forms, there are described in detail herein specific desirable embodiments of the invention. This description is an exemplification of the principles of the invention and is not intended to limit the invention to the particular embodiments illustrated.

For the purposes of this disclosure, unless otherwise indicated, identical reference numerals used in different figures refer to the same component.

The invention disclosed herein is directed to medical implants. The medical implant will be in the form of a device having a flowpath therethrough. Desirably, the implant will be in the form of a stent although it may also be provided in other forms as well, for example, as a vena cava filter.

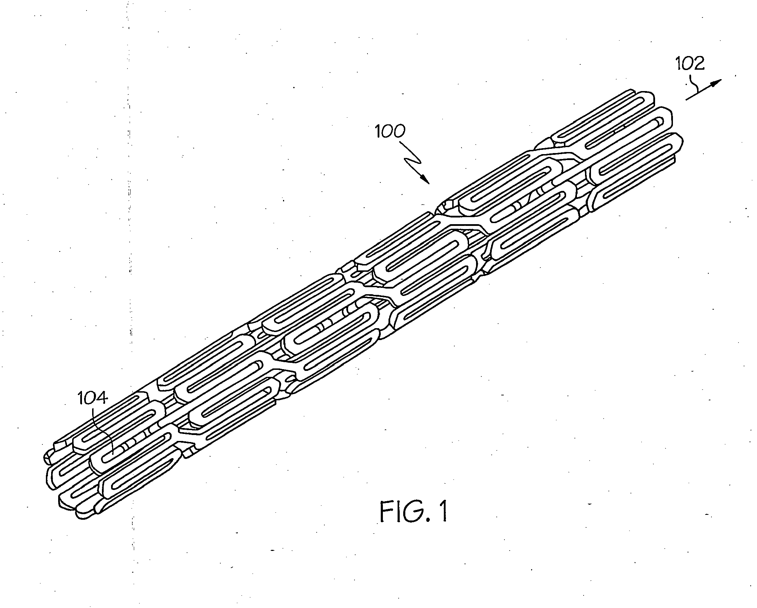

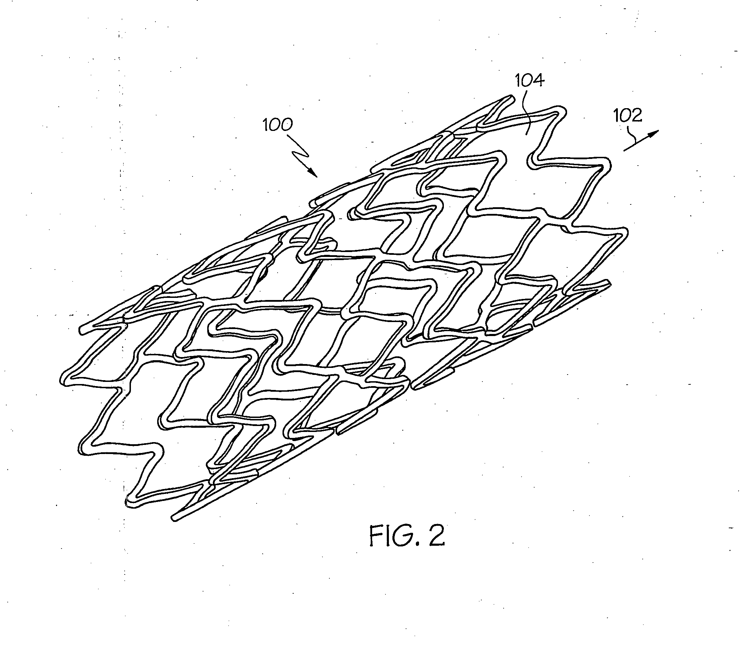

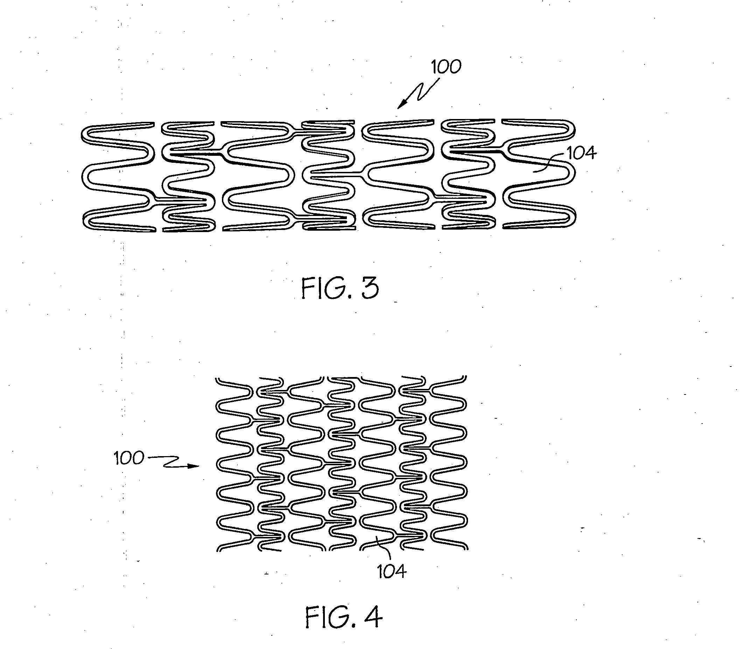

An example of a stent is shown generally in an unexpanded state at 100 in FIG. 1. Stent 100 has a flowpath therethrough in the direction of the longitudinal axis 102 of the stent. The stent has a sidewall with a plurality of openings 104 therethrough. The stent of FIG. 1 is shown in an expanded state in FIG. 2. Another stent design is shown generally at 100 in FIGS. 3 and 4. The stent designs of FIGS. 1-4 are shown for exemplary purposes only. The inventive stents disclosed herein may be manufactured in any other known stent geometry.

The stent may be manufactured using any suitable stent manufacturing technique. The stent may be manufactured from a sheet of metal, from a tube or may be made from individual cylinders which are joined together. In the case of a stent manufactured from a sheet of metal, the sheet may be rolled and the edges of the metal may optionally be secured to another or may be left unsecured to one another. The openings may be provided in the stent by chemically etching the sheet or tube, by laser cutting the tube or through any other known technique for providing such openings.

The invention may also be provided in the form of a vena cava filter. More information concerning vena cava filters may be found in U.S. No. 5,059,205 and in U.S. No. 5,951,585.

The invention is directed to medical implants to medical implants such as those discussed above comprising a tungsten-rhenium alloy. The implant is manufactured from a tungsten-rhenium alloy consisting essentially of tungsten and rhenium. Typically, in the inventive implants, tungsten will be present in an amount ranging from about 75 weight percent to about 99 weight percent and rhenium will be present in an amount ranging from about 25 weight percent to about 1 weight percent. Desirably, if trace impurities are present, each impurity will be present in an amount not exceeding 1 percent by weight.

In one desirable embodiment, the tungsten-rhenium alloy consists essentially of tungsten in approximately 75 weight percent and rhenium in approximately 25 weight percent. In another desirable embodiment, the tungsten-rhenium alloy consists essentially of tungsten in approximately 95 weight percent and rhenium in approximately 5 weight percent. In another desirable embodiment, the tungsten-rhenium alloy consists essentially of tungsten in approximately 97 weight percent and rhenium in approximately 3 weight percent.

The entirety of the implant in general and stent in particular is made of the tungsten-rhenium alloy.

Desirably, stents made from a tungsten-rhenium alloy will have a modulus of elasticity of at least 400 GPa.

In the case of an implant comprising a tungsten-rhenium alloy, the tungsten is desirably present in an amount ranging from about 75 weight percent to about 99 weight percent and the rhenium is desirably present in an amount ranging from about 25 weight percent to about 1 weight percent.

Optionally, the stent may have a modulus of elasticity of 400 GPa or greater. Stents formed of tungsten-rhenium alloy typically have such an elasticity.

The invention is directed to any of the above stents whether in an unexpanded state or in an expanded state.

Any of the inventive stents disclosed above may be provided with a uniform diameter or may taper in portions or along the entire length of the stent. Also, thickness of the various portions of the inventive stents may increase or decrease along a given portion of the stent. For example, one or both ends of the stent may have a thicker wall or a thinner wall than the middle of the stent. The stent may also be provided in an embodiment in which the wall thickness increases along the length of the stent.

The inventive stents disclosed herein may find use in coronary arteries, renal arteries, peripheral arteries including illiac arteries, arteries of the neck and cerebral arteries. The stents of the present invention, however, are not limited to use in the vascular system and may also be advantageously employed in other body structures, including but not limited to arteries, veins, biliary ducts, urethras, fallopian tubes, bronchial tubes, the trachea, the esophagus and the prostate.

In another embodiment, the invention is also directed to vena cava filters and other medical implants formed of the alloys disclosed herein.

Any of the inventive devices disclosed herein may be manufactured from commercially available specimens of the metal or alloy. The metal will usually have to be annealed for a brief period of time. Typically, the annealing will be carried out at a temperature which is approximately one half of the melt temperature of the metal for a period ranging between one and fifteen minutes.

Any of the inventive medical implants disclosed herein may be provided with suitable coatings to render portions of or the entirety of the stent radiopaque and/or with drugs and/or with polymer coatings. For example, the stents may be coated with gold or other noble metals or sputtered with tantalum or other metals. Other radiopaque metals which may be used include platinum, platinum-tungsten, palladium, platinum-iridium, rhodium, tantalum, or alloys or composites of these metals. Some of these metals, however, may impact the MRI compatibility of the stent.

The inventive stents or other medical devices may also be provided with various bio-compatible coatings to enhance various properties of the stent or other medical device. For example, the inventive stents or other medical devices may be provided with lubricious coatings. The inventive stents or other medical devices may also be provided with drugs or drug-containing coatings which release drugs over time.

The inventive stents or other medical devices may also be provided with a sugar or more generally a carbohydrate and/or a gelatin to maintain the stent or other medical device on a balloon during delivery of the stent or other medical device to a desired bodily location. Other suitable compounds for treating the stent include biodegradable polymers and polymers which are dissolvable in bodily fluids. Portions of the interior and/or exterior of the stent or other medical devices may be coated or impregnated with the compound. Mechanical retention devices may also be used to maintain the stent or other medical device on the balloon during delivery. To that end, the use of other coatings on the inventive stents or other medical devices is ,also within the scope of the invention.

The coating may comprise one or more non-genetic therapeutic agents, genetic materials and cells and combinations thereof as well as other polymeric coatings.

Non-genetic therapeutic agents include anti-thrombogenic agents such as heparin, heparin derivatives, urokinase, and PPack (dextrophenylalanine proline arginine chloromethylketone); anti-proliferative agents such as enoxaprin, angiopeptin, or monoclonal antibodies capable of blocking smooth muscle cell proliferation, hirudin, and acetylsalicylic acid; anti-inflammatory agents such as dexamethasone, prednisolone, corticosterone, budesonide, estrogen, sulfasalazine, and mesalamine; antineoplastic/antiproliferative/anti-miotic agents such as paclitaxel, 5-fluorouracil, cisplatin, vinblastine, vincristine, epothilones, endostatin, angiostatin and thymidine kinase inhibitors; anesthetic agents such as lidocaine, bupivacaine, and ropivacaine; anti-coagulants such as D-Phe-Pro-Arg chloromethyl keton, an RGD peptide-containing compound, heparin, antithrombin compounds, platelet receptor antagonists, antithrombin anticodies, anti-platelet receptor antibodies, aspirin, prostaglandin inhibitors, platelet inhibitors and tick antiplatelet peptides; vascular cell growth promotors such as growth factor inhibitors, growth factor receptor antagonists, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; and agents which interfere with endogenous vascoactive mechanisms.

Genetic materials include anti-sense DNA and RNA, DNA coding for, anti-sense RNA, tRNA or rRNA to replace defective or deficient endogenous molecules, angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin like growth factor, cell cycle inhibitors including CD inhibitors, thymidine kinase ("TK") and other agents useful for interfering with cell proliferation the family of bone morphogenic proteins ("BMP"s"),BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Desirable BMP"s are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA"s encoding them.

Cells can be of human origin (autologous or allogeneic) or from an animal Source (xenogeneic), genetically engineered if desired to deliver proteins of interest at the transplant site. The cells may be provided in a delivery media. The delivery media may be formulated as needed to maintain cell function and viability.

Suitable polymer coating materials include polycarboxylic acids, cellulosic polymers, including cellulose acetate and cellulose nitrate, gelatin, polyvinylpyrrolidone, cross-linked polyvinylpyrrolidone, polyanhydrides including maleic anhydride polymers, polyamides, polyvinyl alcohols, copolymers of vinyl monomers such as EVA, polyvinyl ethers, polyvinyl aromatics, polyethylene oxides, glycosaminoglycans, polysaccharides, polyesters including polyethylene terephthalate, polyacrylamides, polyethers, polyether sulfone, polycarbonate, polyalkylenes including polypropylene, polyethylene and high molecular weight polyethylene, halogenated polyalkylenes including polytetrafluoroethylene, polyurethanes, polyorthoesters, proteins, polypeptides, silicones, siloxane polymers, polylactic acid, polyglycolic acid, polycaprolactone, polyhydroxybutyrate valerate and blends and copolymers thereof, coatings from polymer dispersions such as polyurethane dispersions (for example, BAYHDROL^{®}), fibrin, collagen and derivatives thereof, polysaccharides such as celluloses, starches, dextrans, alginates and derivatives, hyaluronic acid, squalene emulsions. Polyacrylic acid, available as HYDROPLUS^{®} (Boston Scientific Corporation, Natick, Mass.), and described in U.S. Pat. No. 5,091,205 is particularly desirable. Even more desirable is a copolymer of polylactic acid and polycaprolactone.

The inventive stents may also be used as the framework for a graft. Suitable coverings include nylon, collagen, PTFE and expanded PTFE, polyethylene terephthalate and KEVLAR, any of the materials disclosed in US 5,824,046 and US 5,755,770 and biograft materials. Examples of biograft materials include, but are not limited to, fibrin, collagen, elastin and hyaluronic acid. More generally, any known graft material may be used including synthetic polymers such as polyethylene, polypropylene, polyurethane, polyglycolic acid, polyesters, polyamides, their mixtures, blends and copolymers.

Suitable stent delivery devices such as those disclosed in US 6,123,712, US 6,120,522 and US 5,957,930 may be used to deliver the inventive stents to the desired bodily location. The choice of delivery device will depend on whether a self-expanding or balloon expandable stent is used. The inventive stents may be delivered in conjunction with one or more stent retaining sleeves. An example of stent retaining sleeves is disclosed in US provisional application 60/238178.

## Claims

1. A medical implant for implantation in a bodily vessel, the implant having a flow passage therethrough, wherein the entirety of the implant except for an optional coating is made of a tungsten-rhenium alloy.

2. The medical implant of claim 1 in the form of a stent.

3. The medical implant of claim 2 wherein the tungsten-rhenium alloy consists of tungsten and rhenium.

4. The medical implant of claim 3 wherein the tungsten is present in an amount ranging from about 75 weight percent to about 99 weight percent.

5. The medical implant of claim 4 wherein the rhenium is present in an amount ranging from about 25 weight percent to about 1 weight percent.

6. The medical implant of claim 2 having a sidewall and a plurality of openings therein, the implant having been made from a sheet of material or from a tube.

## Patentansprüche

1. Ein medizinisches Implantat für die Implantation in ein Körpergefäß, wobei das Implantat eine Durchflusspassage aufweist, wobei die Gesamtheit des Implantats, abgesehen von einer optionalen Beschichtung, aus einer Wolfram-Rhenium-Legierung gemacht ist.

2. Das medizinische Implantat gemäß Anspruch 1 in der Gestalt eines Stents.

3. Das medizinische Implantat gemäß Anspruch 2, wobei die Wolfram-Rhenium-Legierung aus Wolfram und Rhenium besteht.

4. Das medizinische Implantat gemäß Anspruch 3, wobei das Wolfram in einer Menge, die von einem Massenanteil von ungefähr 75 Prozent bis zu einem Massenanteil von ungefähr 99 Prozent reicht, enthalten ist.

5. Das medizinische Implantat gemäß Anspruch 4, wobei das Rhenium in einer Menge, die von einem Massenanteil von ungefähr 25 Prozent bis zu einem Massenanteil von ungefähr 1 Prozent reicht, enthalten ist.

6. Das medizinische Implantat gemäß Anspruch 2, das eine Seitenwandung mit einer Mehrzahl von Öffnungen in dieser hat, und das aus blattförmigem oder röhrenförmigem Material gemacht wurde.

## Revendications

1. Implant médical destiné à l'implantation dans un vaisseau corporel, l'implant présentant un passage d'écoulement à travers lui, la totalité de l'implant, à l'exception d'un revêtement optionnel, étant fabriquée en un alliage de tungstène-rhénium.

2. Implant médical selon la revendication 1, sous la forme d'un stent.

3. Implant médical selon la revendication 2, dans lequel l'alliage de tungstène-rhénium est constitué de tungstène et de rhénium.

4. Implant médical selon la revendication 3, dans lequel le tungstène est présent dans une quantité d'environ 75 % en poids à environ 99 % en poids.

5. Implant médical selon la revendication 4, dans lequel le rhénium est présent dans une quantité d'environ 25 % en poids à environ 1 % en poids.

6. Implant médical selon la revendication 2, ayant une paroi latérale et une pluralité d'ouvertures dans celle-ci, l'implant ayant été fabriqué à partir d'une feuille de matériau ou à partir d'un tube.
